(19) Europäisches Patentamt / European Patent Office / Office européen des brevets

(11) **EP 3 899 522 B1**

(12) **EUROPEAN PATENT SPECIFICATION**

(45) Date of publication and mention
of the grant of the patent:
**03.12.2025 Bulletin 2025/49**

(21) Application number: **19839262.3**

(22) Date of filing: **31.10.2019**

(51) International Patent Classification (IPC):
**G01N 29/04** (2006.01)     **G01M 3/00** (2006.01)
**G01N 27/28** (2006.01)     **F17D 5/06** (2006.01)
**G06N 20/00** (2019.01)     **G01M 3/24** (2006.01)
**G01M 3/28** (2006.01)     **G06N 3/126** (2023.01)
**G06N 5/01** (2023.01)     **G06N 20/20** (2019.01)

(52) Cooperative Patent Classification (CPC):
**G01N 29/04; F17D 5/06; G01M 3/243;**
**G01M 3/2815; G06N 3/126; G06N 5/01;**
**G06N 20/20;** G01N 2291/0258

(86) International application number:
**PCT/IB2019/001277**

(87) International publication number:
**WO 2020/128605 (25.06.2020 Gazette 2020/26)**

(54) **AN IMPROVED METHOD FOR EVALUATING PIPE CONDITION**

VERBESSERTES VERFAHREN ZUR BEURTEILUNG EINES ROHRZUSTANDS

PROCÉDÉ AMÉLIORÉ POUR L'ÉVALUATION DE L'ÉTAT D'UNE CANALISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2018 EP 18306777**

(43) Date of publication of application:
**27.10.2021 Bulletin 2021/43**

(73) Proprietor: **SUEZ International
92800 Puteaux (FR)**

(72) Inventors:
• **CLAUDIO, Karim
08036 Barcelona (ES)**
• **LECLERC, Cyril
33110 Le Bouscat (FR)**

(74) Representative: **Lavoix
2, place d'Estienne d'Orves
75441 Paris Cedex 09 (FR)**

(56) References cited:
**US-A- 4 998 208          US-A1- 2018 300 639
US-A1- 2019 303 791**

**Description**

FIELD OF THE INVENTION

The present invention relates to the field of pipe management for a network of distribution of fluid. More specifically, it relates to the prediction of pipe failure.

BACKGROUND PRIOR ART

Fluids, for example water, oil or gas are usually delivered through large networks that contain a large number of pipes. Over time, the pipes are subject to degradations, for example due to corrosion. Sever pipe conditions may lead to leaks in the network. It is therefore desirable to evaluate the probability of pipe failures, in order to repair or replace damaged pipes before they break or create leaks.

The pipe repair can indeed be tailored according to the probability of failure of each pipe, but also according to the consequences of failures. The consequence of a failure may be dependent upon the importance of a pipe (for example, a failure in a pipe that provides water to an hospital may be considered as especially deleterious), and/or the consequences of the break in the surrounding environment (for example, the failure of an oil pipe may be considered as very detrimental if the pipe is located in a natural reserve). If pipe repair may be subject to different rules, an accurate repair of pipe always requires an accurate estimation of the probability of pipe failure.

A first solution for predicting pipe failures consists in performing terrain measurements of all pipes to evaluate the current degradation of the pipes, and infer from the degradation of the pipe a probability of failure.

In order to do so, pipes can be inspected directly. A number of direct inspection methods exist. For example, the pipes can be inspected visually. Pipes can also be inspected using measurements. For example, an electromagnetic flux can be applied to a pipe and analyzed. It is also possible to perform acoustical measurements that consist in sending acoustical signals and analyzing the response of various portions the pipe to the acoustical signal in order to determine their thickness.

However, such measurements require the displacement of an operator, or local measurements, which limits the number of pipes that can be inspected. Moreover, they allow only an evaluation of the current condition of the pipe, but fail to provide a prediction of a future probability of pipe failure.

Other methods are based on statistical models that allow a prediction of pipe break based on a history of previous pipe breaks, and features of the pipe. However, these methods are inaccurate, because a history of previous pipe breaks is not available for all pipes, and the lack of information relative to the current state of the pipe limit the accuracy of the method.

The documents US 4998208, US 2019/303791 and US 2018/300639 are known but fail to solve the above mentioned problem. US 4998208 discloses a piping corrosion monitoring system which generates inspection dates for individual piping and other elements, such as pressure vessels, in a process plant. The process plant is divided into circuits made up of piping and associated vessels expected to be exposed to a common corrosion environment.

There is therefore the need for a method that allows an accurate estimation of current and future pipe failures in a pipe network, even with a limited history of pipe breaks, and a limited number of inspections of pipes.

SUMMARY OF THE INVENTION

To this effect, the invention discloses a computer-implemented method according to claim 1 comprising: a first step of clustering pipe sections of a pipe network into a number of classes, based on pipe parameters relative to the structure or to the environment of the pipe sections; and, for each class of said number of classes: a second step of extracting a representative sample of pipes sections of the class; a third step of obtaining, for each pipe section sample, one or more pipe condition scores determined by a condition assessment procedure; a fourth step of performing an estimation of one or more pipe condition scores for pipe sections that do not belong to the sample based on said pipe parameters, said estimation being parameterized with the pipe condition scores and pipe parameters of the pipe sections of the sample extracted at the second step; a fifth step of parameterization of a predictive model of probabilities of pipe failures according to pipe condition scores; a sixth step of performing timed predictions of probabilities of pipe failures according to said predictive model.

Such pipe parameters may for example comprise one or more of: structural parameters (length of the pipe; material of the pipe; diameter of the pipe; age of the pipe...), or environmental or surrounding parameters (temperatures of the environment of the pipe; humidity of the environment of the pipe; nearby equipment (for example, presence of a nearby road or airport that may create vibration that diminish the lifespan of the pipe); water quality; pressure; pressure variation; surge; traffic load; ground movement...).

A condition assessment procedure designates any procedure to assess the condition of a pipe using a kind of inspection or analysis of the pipe. Non limitative examples of condition assessment procedures thus include visual inspection, measurements that may be performed on-site (acoustical analysis, electromagnetic analysis...) or be collecting samples and analyzing it in a laboratory. More generally, any kind of human inspection or measurements performed on the pipes that allows assessing the condition of the pipe can be used as a condition assessment procedure. A pipe condition score is a score that characterizes the condition of a given pipe section. A pipe condition score can be expressed using various scales. For example scales providing an indicative state of the pipe (such "good", "average", "poor"...), normalized scales between 0 and 1, or a measurements indicative of the pipe condition (such as pipe thickness) may be used.

The first step groups the pipe sections into a number of classes, and, within each class, the pipe sections are affected with a substantially similar degradation process.

As the condition assessment procedures of pipes are long and costly, the objective of the second step is to provide, for each class, a limited number of pipe sections that are as representative as possible of the pipe sections of the class, so that the condition assessment of a limited number of pipe sections in the class allows extracting general rules for estimating pipe conditions of pipe sections of the class based on pipe parameters. The condition assessment procedures provide, for the selected pipe sections, reliable pipe condition scores, because they rely on an inspection, observations or measurement performed on the actual pipe.

The fourth step consists in performing an estimation of pipe condition for the pipe sections for which no condition assessment procedure has been conducted. Since the pipe sections are grouped into classes of pipes affected by substantially similar degradation processes, and a condition assessment procedure has been conducted for some pipe sections within each class, the fourth step is accurately parameterized, and provides a reliable estimation of pipe condition.

The fifth step allows defining values of parameters of predictive model of pipe failures based on pipe condition scores. This allows obtaining accurate predictions of pipe failures depending on time because, as an accurate pipe condition score is available for each pipe, the timed predictions of pipe failure can be adjusted to the current condition, and probability of failure of each pipe.

The method therefore allows performing timed predictions of probabilities of pipe failures that are accurate for each pipe of the pipe network, with a limited number of pipe inspections.

The method therefore provides a very powerful tool for planning pipe inspections and renewal.

Advantageously, the predictive model is a probabilistic model that predicts the probability of failure of a pipe as a function of at least a history of past failures, the age of the pipe, and external factors.

The external factors represent each parameter other than history of past failure, and age of the pipe, that has an impact on the probability of failure. Such a model therefore provides the advantage of being adaptable to the available parameters, because parameters can be integrated as external factors according to their availability.

Advantageously, the pipe condition scores are integrated as external factors.

This allows an efficient integration of the probability of failure within existing models.

Advantageously, the predictive model of probabilities of pipe failures consists in a combination of: a predictive function of probabilities of failures according to time parameterized with said at least one parameters; an instantaneous probability of failure determined based at least on pipe conditions scores of each pipe.

The predictive model thus provides accurate time-based probabilities of pipe failures, because the predictive function provides a good estimation of the evolution of probabilities in time, while the instantaneous probability of failure determined based on pipe condition scores provide good estimations of the probability of failure of a pipe at the current time.

Advantageously, said number of classes is a predefined number of classes, and the first step comprises the application of a Gaussian Mixture Model (GMM) to the pipes for clustering the pipe sections into said predefined number of classes.

This allows grouping the pipe sections into classes having members with homogeneous features, thus allowing an efficient clustering of the pipe sections.

Advantageously, the second step of extracting the sample comprises: a seventh step of initializing a set of candidate samples of pipe sections; an eighth step of iteratively modifying said set of candidate samples using: a genetic algorithm based on an objective function comprising a minimization of the difference of average pipe parameters of the pipe sections of the sample, and the average pipe parameters of the pipe sections of the class; a ninth step of selecting the candidate sample that optimizes said objective function.

The genetic algorithm optimizes, over successive iterations, the objective function that consists in minimizing the differences between average parameters of the sample and the class. The genetic algorithm provides the advantage of allowing an optimization the function while testing samples comprising very different combinations of pipe sections. This therefore allows obtaining samples whose average pipe parameters are as close as possible to the average pipe parameters of the corresponding classes. The samples thus provide a good representation of the classes, and reliable models of the classes can be built from these samples. Moreover, genetic algorithms have a limited computational complexity.

Advantageously, the relative size of each sample is negatively correlated with the relative homogeneity of each corresponding class.

In general, the more homogenous a class is, the lower the size of the sample needs to be to model accurately the degradation behavior of pipe sections of the class. Indeed, a lower number of training values are necessary to train a model, if the members of the class are homogeneous. Therefore, negatively correlating the relative sizes of the samples with the relative homogeneities of the classes allows allocating a size of samples to optimize the reliability of the predictions for all classes for a given number of pipe condition procedures to conduct.

Advantageously, the condition assessment procedure of the third step is chosen in a group comprising one or more of: an analysis of an electromagnetic flux applied to the pipe section; an acoustical analysis of the pipe section; the extraction, and analysis in a laboratory of a sample of the pipe section; and wherein each of the condition assessment procedure provides pipe condition scores at the same scale.

This allows selecting the most adapted measurement or observation method for each pipe section, and comparing easily the measurements or observations between the pipe sections.

Advantageously, the condition assessment procedures provide two or more pipe condition scores corresponding to different parts of pipe sections and chosen in a group comprising: an inner coating condition score; an outer coating condition score; a joint condition score.

This allows evaluating the evolution of the condition of different parts of pipe sections. This is useful, because the condition of different parts of the pipes can evolve in different ways depending on the structural and environmental pipe parameters.

Advantageously, a single pipe condition score is obtained from the two or more pipe condition scores corresponding to different parts of pipe sections, using a weighted or orthogonal sum.

This allows summarizing the pipe condition within a single pipe condition score that takes into account the specific degradations of different parts of the pipe.

Advantageously, the one or more pipe condition scores are associated with one or more reliability indexes.

This allows taking into account the reliability of each measurement in order to model as efficiently as possible the evolution of pipe conditions.

Performing an estimation of one or more pipe condition scores for pipe sections that do not belong to the sample comprises: training, for a class, a supervised machine learning engine that predicts pipe condition scores based on pipe parameters using pipe sections that belongs to the sample; using said supervised machine learning engine to predict pipe condition scores based on pipe parameters of the pipe sections of the class that do not belong to the sample.

This allows an efficient modeling of the pipe conditions, because the pipes have been previously clustered into classes of similar pipe sections, and a model is trained for each class. The model is therefore well adapted to the pipe sections on which it will be applied.

Advantageously, said supervised machine learning engine is a random forest machine learning engine.

A random forest algorithm is especially well suited for this task. Indeed, a random forest removes the pipe parameters that are not predictive of pipe conditions. This is especially effective here, because a large number of structural or environmental parameters may be used. The random forest algorithm automatically uses only the parameters that actually allow predicting the pipe condition.

The invention also discloses computer program product, stored on a non-transitory computer-readable medium, said computer program product comprising code instructions for executing a method according to an aspect of the invention.

The invention also discloses a device comprising a processor configured to execute a method according to an aspect of the invention.

BRIEF DESCRIPTION OF THE DRAWINGS

The invention will be better understood and its various characteristics and advantages will emerge from the following description of a number of exemplary embodiments and its appended figures in which:

- Figure 1 displays a network of pipes on which a method according to the invention may be implemented;
- Figure 2 displays a computer-implemented method according to the invention;
- Figure 3 displays an example of clustering of pipes in an embodiment of the invention;
- Figure 4 represents an example of parameterization of a predictive model of probabilities of pipe failures according to pipe condition scores in a number of embodiments of the invention;
- Figure 5 represents the relationship between predicted and actual pipe failures, with a model respectively with and without using pipe condition scores.

DETAILED DESCRIPTION OF THE INVENTION

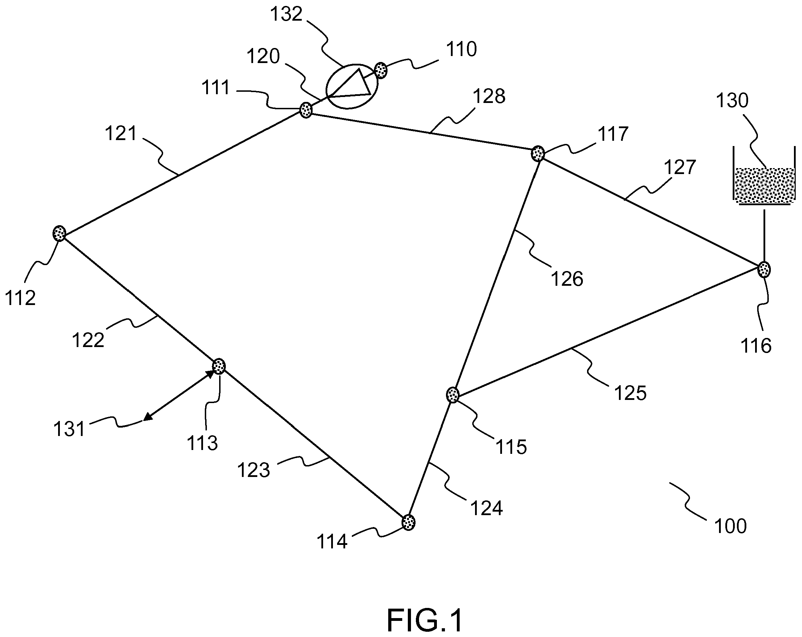

Figure 1 displays a network of pipes on which a method according to the invention may be implemented.

The network 100 displayed on figure 1 represents a water distribution network and comprises a plurality of nodes 110, 111, 112, 113, 114, 115, 116 and 117, and a plurality of arcs 120, 121, 122, 123, 124, 125, 126, 127 and 128. The nodes typically represent the connections with water sources or water reservoirs, for example the reservoir 130 at node 116, the connections with the user of the water distribution system, for example consumption 131 at node 113 and the connections between the arcs. The arcs represent pipes between the nodes. The network can be equipped with equipment such as valves and pumps. A pump 132 is for example present on the arc 120. More generally, a node can be a junction between two or three pipes, a point at which inputs or outputs of the network are found, for example a point where a user consumes water, or a point at which water is injected in the network. A node can also represent a sub-network, for example a neighborhood grouped under a single node.

The exemplary network of figure 1 has a low number of pipes. However, typical water network has a much higher number of pipes. For example, the invention may be applied to pipe networks comprising 10,000, or even more, km of pipes. For example, the invention can be applied to pipe networks representing a metropolitan area. For example, the drinking water distribution network of Paris area comprises around 37,000 km of pipes; the drinking water distribution network of Tokyo area comprises around 27,000 km of pipes; the drinking water distribution network of London area comprises around 21,000 km of pipes; the drinking water distribution network of Adelaïde area comprises around 9,000 km of pipes; the drinking water distribution network of Casablanca area comprises 4,500 km of pipes. The total length of drinking water pipes in the European Union is estimated to be around 4,230,000 km.

Each pipe of the network may be progressively degraded over time, and break when the degradation becomes too important. An objective of the invention is thus to obtain a reliable estimation of pipe condition and probability of breaks in the future in order to plan pipe inspection, repair and renewal and as efficiently as possible.

Although figure 1 displays a water distribution network, the invention may be applied to other kinds of fluid networks, such as oil or gas distribution networks.

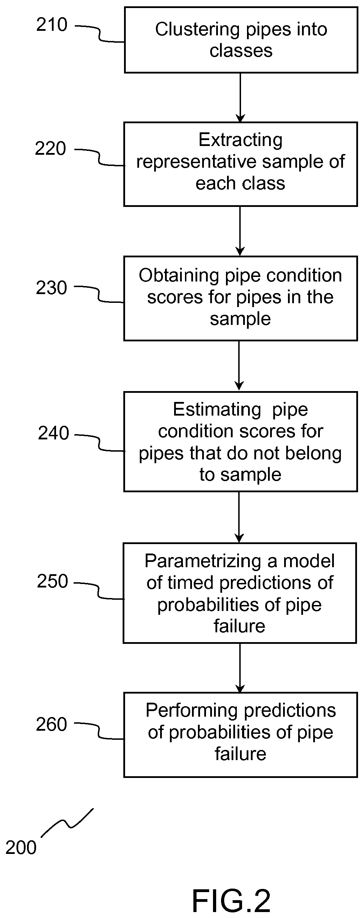

Figure 2 displays a computer-implemented method according to the invention.

One of the objectives of the computer-implemented method according to the invention is to determined time-based probabilities of failures of pipes in a pipe networks, that is to say be able to predict, for any pipe of a pipe network, a reliable probability of failure at a given time.

The method 200 applies to a pipe network, that may be for example a water, oil or gas distribution network. The network is formed of interconnected pipes. The pipes are split into sections. A pipe section may be either an entire pipe, or a part of a pipe. It may be useful to split large pipes into a plurality of sections, because the section may be located into different environments, and thus pipe condition may evolve differently over time for the various sections of the same pipe. Conversely, adjacent and interconnected pipes may be grouped within the same pipe section, if they share certain properties (for example, the same material, thickness, etc.). Pipe sections may be defined according to different rules. For example, the pipes may be split into sections:

- according to changes in features of the pipes or environment changes (i.e., a pipe can be split in sections if the material of the pipe changes, if there is a significant modification of the environment over the length of the pie, etc.);
- graphically, by a user defining manually the limits of pipe sections;
- to reflect a maximum length of a single pipe section.

The method 200 can be implemented within an asset management solution that allows controlling the condition of pipes in a network, predicting the probabilities of failures of pipes, and planning pipe maintenance and renewal. Such a solution may also allow the management of other assets of the network, such as reservoir, pumps, etc.

An asset management solution has access to one or more data storages that store information relative to the assets. For example, this may grant access to values of a number of pipe parameters. Such pipe parameters may for example comprise one or more of:

- structural parameters:

  - length of the pipe;
  - material of the pipe;
  - diameter of the pipe;
  - age of the pipe;

- environmental or surrounding parameters:

- temperatures of the environment of the pipe;
- humidity of the environment of the pipe;
- nearby equipment (for example, presence of a nearby road or airport that may create vibration that diminish the lifespan of the pipe);
- water quality;
- pressure;
- pressure variation;
- surge;
- traffic load;
- ground movement;
- ...

These parameters have an influence on the evolution of pipe condition, and the probability of pipe failure over time. For example, a pipe that is subject to extreme temperature, high humidity, or the vibration created by a nearby infrastructure will be subject to a faster degradation. The degradation rate also depends on structural parameters of the pipes, for example the material and diameter of the pipe. Certain parameters may also interact. For example, the impact of humidity on the pipe may be dependent upon the material of the pipe.

In a number of embodiments of the invention, the values of pipe parameters can be retrieved, for each pipe section, using some kind of storage or database.

The method 200 comprises a first step 210 of clustering pipe sections of a pipe network into a number of classes, based on pipe parameters.

This first step 210 consists in grouping the pipe sections that share similar parameters within the same class. The pipe sections that belong to the same class are thus likely to evolve in a similar way over time.

This can be performed in different ways. Unsupervised machine learning algorithms are especially well suited for clustering the pipes. Clustering can be performed based on any combination of pipe parameters. For example, clustering can be performed according to one or more parameters expected to have an impact on pipe degradation: material, diameter, length, pressure, water quality, ground quality, stay current, road traffic, etc... Clustering can be performed to split the pipe sections into a target number of classes, for example a target number of classes predefined by an operator. For example, a target number of four classes can be used. The number of classes can also be adapted to avoid having classes with a very low number of members, which may not be very significant. The number of classes can also be adapted to the size of the network: for example, a lower number of classes can be used for small networks (i.e., networks having a total length of pipes equal to or lower than a predefined threshold). Classes can also be merged a posteriori. For example, a class with a low number of members can be merged with a class having similar features.

Different models or algorithms can be used to perform the clustering. For example, a Gaussian Mixture Model (GMM) model provides good clustering results, because it allows obtaining classes having homogenous members.

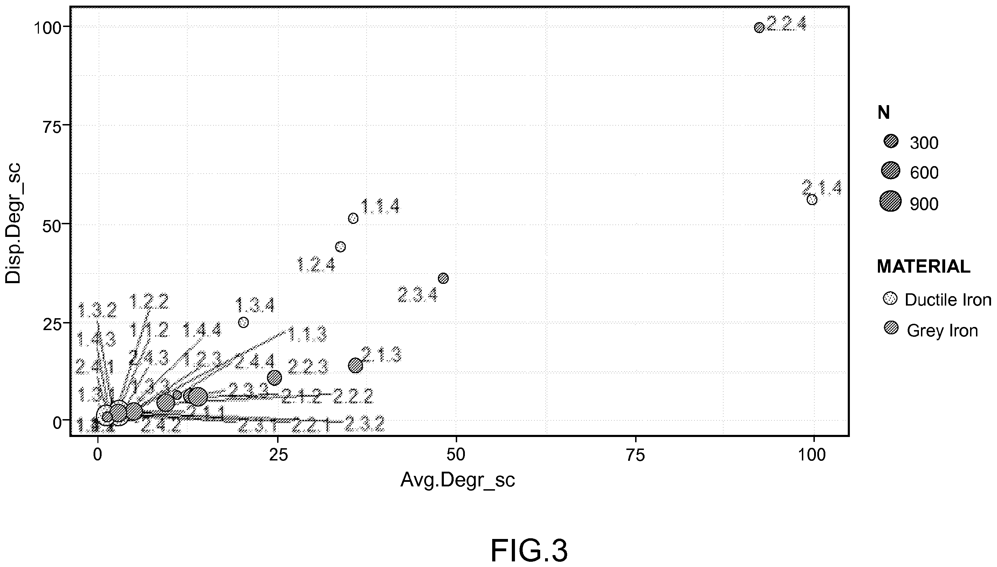

Figure 3 displays an example of clustering of pipes in an embodiment of the invention.

The pipes have been clustered according to a plurality of variables including pipe material and diameter.

The table below show some characteristics of each of the clusters represented in figure 3 (number of pipes, total length of the pipes, and average degradation indicator of the pipes). The average degradation indicator is, in this example, a value between 0 and 1 that indicates the average degradation of pipes in the cluster. It is calculated based on the pipes that have been inspected, and the higher it is, the more degraded pipes are.

*Table 1 - Statistics relative to clusters of pipes*

| *Cluster* | *Number of pipe* | *Total Length (km)* | *Average Degradation Indicator* |
|---|---|---|---|
| 1.1.1 | 1020 | 13,0638 | 0,00073396 |
| 1.1.2 | 2258 | 135,3849 | 0,0024462 |
| 1.1.3 | 815 | 120,05081 | 0,0064775 |
| 1.1.4 | 166 | 27,93472 | 0,05668984 |
| 1.2.1 | 530 | 7,21911 | 0,00052549 |
| 1.2.2 | 789 | 62,2254 | 0,00194201 |
| 1.2.3 | 284 | 59,06881 | 0,00553672 |
| 1.2.4 | 47 | 10,98917 | 0,03270694 |
| 1.3.1 | 382 | 28,36028 | 0,00056146 |

(continued)

| Cluster | Number of pipe | Total Length (km) | Average Degradation Indicator |
|---------|----------------|-------------------|-------------------------------|
| 1.3.2 | 80 | 23,96238 | 0,00243233 |
| 2.1.1 | 782 | 9,87718 | 0,00612382 |
| 2.1.2 | 1254 | 86,93611 | 0,02006067 |
| 2.1.3 | 510 | 73,66109 | 0,05917312 |
| 2.1.4 | 148 | 31,74353 | 0,18194432 |
| 2.2.1 | 668 | 17,24092 | 0,00573948 |
| 2.2.2 | 306 | 42,75907 | 0,02044657 |
| 2.2.3 | 83 | 27,5024 | 0,0816995 |
| 2.3.1 | 251 | 11,7555 | 0,00253111 |
| 2.3.2 | 89 | 17,10164 | 0,00913077 |
| 2.3.3 | 33 | 12,83664 | 0,02550907 |
| 3.1.1 | 469 | 24,27088 | 0,00239257 |
| 4.1.1 | 50 | 9,86144 | 0,0112026 |

Coming back to figure 2, the method 200 comprises a second step 220 of extracting, for each class, a sample of pipe sections of the class.

This step consists in identifying, for each class, a number of pipe sections to inspect. As will be explained in more details hereinafter, these identified pipe sections will be inspected on-site to obtain a pipe condition score, in order to be able to estimate pipe condition scores from pipe parameters within each class. The second step 220 provides, for each class, a limited number of pipe sections that are as representative as possible of the pipe sections of the class.

The pipe sections of the sample may be selected according to a number of techniques and constraints.

According to various embodiments of the invention, the sample can be set to comprise a maximum number or percentage of pipe sections of the class. For example, the sample may comprise twenty pipe sections among a hundred pipe sections that belong to a class. This maximum number or percentage may be defined in different ways. For example, it may be defined by an operator, because it corresponds to a number of pipes that is expected to be sufficient to allow a good estimation of the pipe condition scores, and/or due a maximum number of pipe sections that matches a defined budget for pipe inspection.

In a number of embodiments of the invention, a total budget for pipe inspection, corresponding to a defined number of pipe condition assessment procedures, is allocated among the different classes. This number of pipe conditions procedures can be split between the different samples. The sizes of the samples can be defined according to the relative sizes and homogeneity of the classes. Indeed, a lesser number of pipe inspection procedures are required to provide a reliable modeling of homogeneous class. Thus, a lower size of the sample can be allocated to more homogeneous classes.

The applicant has filed, in the domain of real-time estimation of fluid consumption, an international patent application published under n° WO 2014/060655. The method of estimation of fluid consumption relies on the classification of users into classes of users having similar consumption profiles, the real time measurement of consumption of a sample of users for each class, and the estimation a total consumption of the network based on these partial real time measurements.

This patent application faces the same problem allocating an optimal number of users to different classes in order to have the best global estimation with a limited number of real time measurements. In order to solve this issue, the applicant has defined a formula to define the sizes of the samples depending of the total target size of the samples, the size and dispersion of each class. This formula is provided p. 7 l. 15-28 of the said international publication. This formula can be applied mutadis mutandis to the allocation of the sizes of the samples of pipe sections of the invention, based on the relative sizes and dispersion of values of pipe parameters in the invention. The dispersion may here be the dispersion of the pipe conditions scores, or more generally a degradation indicator of each pipe that may be calculated based on past failures, or the same parameters than the pipe condition scores. Here, the sizes of the samples allows an efficient modeling of each class, because the classes with more heterogeneous pipes will be modeled using a relatively higher number of pipe condition assessments.

In a number of embodiments of the invention, the sample comprises pipe sections, for which an inspection has been previously planned. This may be for example pipe sections that are already expected to be already in a critical state, and/or that are planned to be inspected by the inspection plan of the operator. This allows saving pipe inspection budget, because the inspection that are already planned will be used to feed the sample.

In a number of embodiments of the invention, a sample comprises randomly selected pipe sections. This provides the advantage of being simple, but fails to ensure that the selected pipe sections are fairly representative of the class.

In a number of embodiments of the invention, a sample comprises pipe sections that are expected to be the most representative of the class. This allows obtaining a sample that provides, for a given number of pipes, the best estimation of pipe condition based on pipe parameters. The pipe sections can be expected to be the most representative of the class, for example if their pipe parameters are as close as possible to the average of pipe parameters within the class. This can be performed for each pipe section individually, by ensuring that the parameters of each selected pipe section are individually as close as possible to the average of the pipe sections of the class.

It can also be performed for the sample as a whole, by ensuring that the average of the parameters of the pipes of the sample are as close as possible to the average of the parameters of the pipes. This presents the advantage of ensuring that the pipe sections of the sample are, as a whole, representative of the pipe sections of the class. It also allows selecting a sample of pipe sections representative of the class, even if the selection some pipe sections of the samples is constrained (for example because some pipe sections are mandatorily selected because they belong to the pipe inspection plan of the operator). In a number of embodiments of the invention, the pipe sections of the sample are selected using a genetic algorithm. This consists in initializing a set of candidate samples, then iteratively modifying this set using selection, crossover and mutation, in order to iteratively obtain better candidate samples.

Genetic algorithms use an objective function that allows ranking candidate samples in order to select the best candidates. In a number of embodiments of the invention, the objective function comprises a minimization of a difference between the average of pipe parameters of the sample, and the average of pipe parameters of the class. Thus, over successive iterations, the candidate pipe samples become more representative of the class.

According to various embodiments of the invention, all the pipe sections of the sample can be modified at each iteration, or some pipe sections are kept in all samples (for example, pipe sections that will be inspected anyway in the pipe inspection plan of the operator). The genetic algorithm provides the ability of obtaining sample of pipes that are representative of the class, even with constraints relative to the selection of pipe sections.

Finally, the candidate sample that optimizes the cost function, that is to say the candidate sample that is considered as the most representative of the class, is selected.

The method 200 further comprises a third step 230 of obtaining, for each pipe section in each sample for each class, one or more pipe condition scores determined by a condition assessment procedure.

As explained above, the pipe sections that are selected for being part of a sample are inspected. Different condition assessment procedures may be used. For example, the pipe conditions may be assessed using:

- an electromagnetic flux;
- an acoustical analysis;
- the extraction of sample of the pipes, that are being analyzed in a laboratory;
- visual inspection;
- CCTV and computer vision;
- tomography;
- drone inspection;
- ...

The condition assessment procedure may be the same for all the pipe sections, or may be selected specifically for each pipe section. The selection of a condition assessment procedure may be performed, depending on the technical feasibility of each method in the environment of the pipe section, or because a procedure is particularly well suited for a given pipe section. For example, metallic pipes can be inspected with electromagnetic flux or acoustic techniques, while non-metallic pipes can be inspected by collecting a sample and sending it to a laboratory. The inspection technique can also be chosen according to the criticality of a pipe, defined by the operator. For example, a pipe section that serves an hospital, or a city center, can be considered as more critical. Thus a more accurate pipe condition assessment procedure, or a procedure that provides more information about the pipe, such as sample collection and laboratory analysis, can be chosen, even if it is more expensive.

The pipe inspection procedure can also be chosen according to its technical feasibility. For example, acoustical inspection is an inexpensive method that does not require to open a trench to reach the pipe, but current acoustical inspection can be performed only on metallic pipes of less than two hundred meters long. An acoustical inspection can thus be performed each time it is technically possible, and if it is not, other methods which are more expensive may be used.

Each of these condition assessment procedures allows providing one or more pipe condition scores of the pipe section that has been inspected. The pipe condition score indicates a level of degradation of the pipe section. When different procedures are used, their output may be expressed using different scales. For example, an acoustical analysis provides a residual thickness of a pipe (or, conversely, a value or percentage of lost thickness), while a laboratory test of a sample of a pipe provides a number of measurements of the pipe such as a corrosion presence and type of corrosion for internal and/or external corrosion, residual thickness, obstruction level, a graphitization level, etc.

In order to obtain homogeneous and comparable measurements the pipe condition scores can be transformed to a normalized scale, so that different inspection methods provide a pipe condition score using the same scale, and all the pipe conditions scores can be compared. For example, pipe condition scores ranging from 0 to 1 can be used, wherein 0 corresponds to a perfect condition, and 1 a failure condition. Thus, each pipe section can be inspected using the most relevant procedure (depending on pipe section characteristics and/or the feasibility of different inspection methods), while the output of the procedures can be compared. For example, if an acoustical analysis is used, the residual thicknesses can be transformed into homogeneous values between 0 and 1; in laboratory tests are used, scores relative to the different analysis parameters can be weighted according to their relative importance.

The pipe condition scores may also belong to a finite number of states, for example 3 states: "good", "average" or "poor".

In a number of embodiments of the invention, a plurality of condition scores are obtained for each pipe, which correspond to different parts of the pipe section. For example, the observation can generate a vector of 3 pipe conditions scores for a pipe section:

- an inner coating condition score;
- an outer coating condition score;
- a joint condition score.

This allows evaluating the evolution of the condition of different parts of pipe sections. This is useful, because the condition of different parts of the pipes can evolve in different ways depending on the structural and environmental pipe parameters. Using a vector of pipe condition scores that correspond to various parts of the pipe section therefore allows taking into account the degradation of different parts of the pipe.

The pipe condition scores or the vector can then be summed using an orthogonal or weighted sum, in order to obtain a single pipe condition score for the pipe section. This allows summarizing the pipe condition within a single pipe condition score that takes into account the specific degradations of different parts of the pipe.

In a number of embodiments of the invention, the pipe condition scores are associated with one or more reliability indexes. The reliability indexes indicate how precise and reliable the observed pipe condition scores are. For example, the reliability indexes may depend upon the observation method. For example, it can depend upon the reliability of the instrument that performed the measure. A measure using instrument can also be considered as more reliable than visual observation.

According to a number of embodiments of the invention, when a vector of a plurality of pipe condition scores corresponding to different parts of a pipe section is obtained, there may be either a single reliability index for the whole vector, or a reliability index for each pipe condition in the vector. Using a reliability index for each pipe condition in the vector allow tailoring the indication of reliability of the measures or observations, when the accuracy of an observation methods is variable for the different parts of the pipe. For example, a visual observation is expected to be more reliable for evaluating the condition of the outer coating than the condition of inner coating of the pipe. Pipe condition scores resulting from visual observations can thus be associated with average reliability index for outer coating, but low reliability index for inner coating.

The method 200 further comprises a fourth step 240 of performing an estimation of one or more pipe condition scores for pipe sections that do not belong to a sample based on said pipe parameters, said estimation being parameterized with the pipe condition scores and pipe parameters for the pipe sections in the samples.

This step consists in training a model, for each of the classes, based on the pipe condition scores that are obtained through pipe condition assessment procedures, then using the model for estimating pipe conditions scores of pipes that were not subject of a condition assessment procedure.

This process allows an efficient modeling of the pipe conditions, because the pipes have been previously clustered into classes of similar pipe sections, and a model is trained for each class. The model is therefore well adapted to the pipe sections on which it will be applied.

This step may be performed in different ways. For example, a statistical model of linear regression can be trained, for each class, by fitting the pipe parameters to the pipe condition scores, for the pipe sections whose conditions have been assessed.

According to the invention, this is achieved by training a supervised machine learning model with the pipes whose conditions have been assessed. Thus, a machine learning engine can be fed, for each class, with the pipe parameters and pipe condition scores of the pipes sections that have been inspected, then used to predict pipe condition scores based on pipe parameters for the pipe sections that have not been inspected.

A random forest algorithm is especially well suited for this task. Indeed, a random forest removes the pipe parameters that are not predictive of pipe conditions. This is especially effective here, because a large number of structural or environmental parameters may be used. The random forest algorithm automatically uses only the parameters that actually allow predicting the pipe condition.

The method 200 therefore allows an accurate estimation of the state of each pipe in a large pipe network, while performing inspection of only a limited number of pipes, which allows saving money and time. Moreover, it allows at any time to obtain a reliable estimation of the state of each pipe in the network.

The method 200 further comprises a fifth step 250 of parameterization of a predictive model of probabilities of pipe failures according to pipe condition scores.

The predictive model of probabilities of pipe failures is a model which is able to calculate a current or future probability of failure of the pipes. The model is thus able to determine, for a given pipe, a probability of failure at a time t, which is the current time or a time in the future.

The use of a pipe condition score for parameterizing the model allows adapting the predictions of failure to the current probability of failure of the pipe. As the previous steps allows obtaining a reliable value of pipe condition for each pipe of the network, the predictive models can be parameterized to provide an reliable value of pipe condition for each pipe, using a limited number of inspection of the pipes.

According to the invention, different models may be used, such as a stochastic model (Yule model, Poisson process, Cox model, etc...), or a machine learning model (for example random forest, or neural network).

More generally, any model that is able to predict a probability of failure of a pipe, at the current time or in the future may be used.

In a number of embodiments of the invention, the predictive model of probabilities of pipe failures predicts the probability of failure of a pipe according to values of at least one parameter chosen in a group comprising:

- parameters characterizing the pipe: length, diameter, structure, material, depth, diameter of the pipe, age, number of past failures, etc.;
- parameters characterizing the operation of the pipe: minimum/maximum pressure of the network, water quality, water speed, etc.;
- parameters characterizing the environment or surrounding of the pipe: quality of soil, climate conditions, presence of traffic/vibration nearby, etc.

The environment or surrounding of the pipe can, according to various embodiments of the invention, encompass, as defined above, any parameter of the immediate environment/surrounding of the pipe, that has an impact on the pipe condition: temperatures of the environment of the pipe; humidity of the environment of the pipe; nearby equipment (for example, presence of a nearby road or airport that may create vibration that diminish the lifespan of the pipe); water quality; pressure; pressure variation; surge; traffic load; ground movement... More generally, parameters characterizing the environment or surrounding of the pipe may be any parameter having an impact on the degradation of the pipe, and that is not related to intrinsic features of the pipe.

Using such parameters allows estimating the probability of failure of each pipe according to its specificities.

In a number of embodiments of the invention, the predictive model is a probabilistic model that predicts the probability of failure of a pipe as a function of at least a history of past failures, the age, and external factors. For example, the LEYP (Linear Extension of Yule Process) model, described in Claudio, K., Couallier, V., & Le Gat, Y. (2014). Integration of time-dependent covariates in recurrent events modelling: application to failures on drinking water networks. Journal de la Société Française de Statistique, 155(3), 62-77, provides a very complete and accurate solution to estimate the probability of failure of a pipe in the future. The LEYP model is based on 3 factors:

- The Yule factor: impact of past failures;
- The Weibull factor: impact of age;
- The Cox factor: impact of "external" factors.

Moreover, the LEYP model is adaptable to a wide range of different parameters, and can thus be used according to the values of parameters that are currently available for a pipe. For example, the model may also take into account some or all of the parameters characterizing the pipe, the parameters characterizing the operation of the pipe, and the parameters characterizing the surrounding or environment of the pipe for which values are available.

According to various embodiments of the invention, the pipe conditions scores may be used to parameter the predictive model in a number of different ways.

For example, if the predictive model predicts the probability of failure of a pipe as a function of at least a history of past failures, the age, and external factors, the pipe condition score can be integrated as an external factor. It can for example be integrated within the Cox factor of the LEYP model. This allows an efficient integration of the probability of failure within existing models.

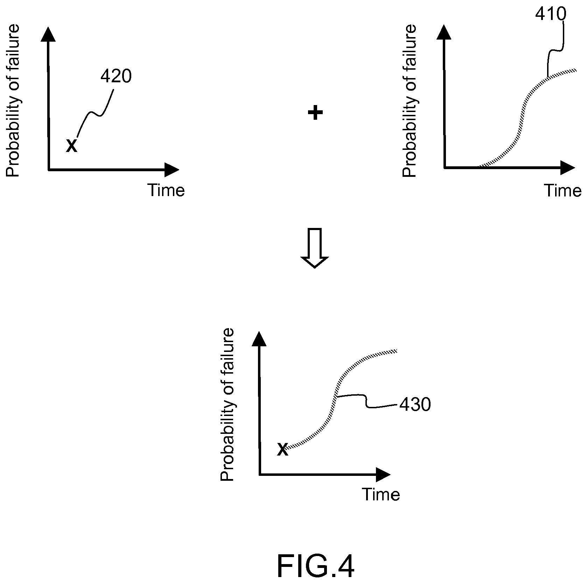

Figure 4 represents an example of parameterization of a predictive model of probabilities of pipe failures according to pipe condition scores in a number of embodiments of the invention.

In a number of embodiments of the invention, the predictive model allows a determination of the probability of failure of a pipe as a function of time 410 depending on a number of parameters that may for example be the examples disclosed above.

In addition, an instantaneous probability of failure 420 is calculated based on the pipe condition score for the pipe. Indeed, the probability of failure of the pipe is dependent upon the pipe condition score: the more degraded the pipe is, the higher the probability of failure is.

The instantaneous probability of failure 420 is then combined with the function of time 410, to obtain a function 430 indicating the probability of failure according to time. For example, the instantaneous probability of failure 420 can be added to the function 410 to obtain the function 430. The function 430 therefore takes into account a number of parameters characterizing the pipe, the environment of the pipe and the operation of the pipe, but also the current condition of the pipe. This therefore provides a much more accurate prediction of the probability of pipe failure than existing models that rely only on pipe, environmental and/or operational parameters.

The model 430 thus provides an accurate time-based probabilities of pipe failures, because the function 410 provides a good estimation of the evolution of probabilities in time, while the probability of failure 420 provides a good estimation of the probability of failure of a pipe at the current time.

One example of such combination is provided by the integration of a pipe condition score within the LEYP model. The LEYP model, explained in details by Renaud, E., Le Gat, Y., & Poulton, M. (2012). Using a break prediction model for drinking water networks asset management: From research to practice. Water Science and Technology: Water Supply, 12(5), 674-682, is based on an intensity of failure at a time t, expressed as the age of the pipe, for a pipe noted $\lambda(t)$, and equal to:

$$\lambda(t) = (1 + \alpha N(t-))\delta t^{\delta-1} exp(\beta_0 + \beta_1 X_1 + \beta_1 X_1 + \cdots + \beta_N X_N)$$

*(Equation 1)*

Wherein:

- $N(t-)$ represents the number of bursts that occurred in the pipe before time t;
- $\delta t^{\delta-1}$ represents the effect of the age of the pipe;
- $X_1, X_2 ... X_N$ represents external parameters.
- $\alpha, \beta_0, \beta_1 ... \beta_N$ represent the parameters of the model.

Several features can be integrated within the external parameters. For example, the following features may be used:

- $X_1$ : length of the pipe;
- $X_2$ diameter of the pipe;
- $X_3$ : pressure of the pipe;
- $X_4$ : soil aggressiveness;
- $X_5$ : pipe condition score, as calculated by step 240, in a scale ranging from 0 (no degradation) to 1 (completely degraded pipe).

The values of the parameters $\alpha, \beta_0, \beta_1 ... \beta_N$ may be obtained in a number of different ways. For example, they may be obtained through the optimization of a Maximum Likelihood Estimation.

In the LEYP model, the probability of failure, and the number of expected failure are calculated based on a "baseline intensity", which is interpreted as the intensity of the first failure (N(t-) = 0):

$$\lambda_0(t) = \delta t^{\delta-1} exp(\beta_0 + \beta_1 X_1 + \beta_1 X_1 + \cdots + \beta_N X_N)$$

*(Equation 2)*

Then the probability that at least one failure occurs in a period [C;D], knowing that a number m of bursts occurred in a period [A;B] (A, B, C, D being times, expressed as ages of a pipe) is defined as:

$$P\{X_{|C-D|} > 0 \big| m_{|A-B|}\} = \left(\frac{\mu_B - \mu_A + 1}{\mu_D - \mu_C + \mu_B - \mu_A + 1}\right)^{\frac{1}{\alpha} + m}$$

*(Equation 3)*

Wherein $\mu$ is calculated as:

$$\mu_t = exp\left(\alpha \int_0^t \lambda_0(u)du\right)$$

*(Equation 4)*

The LEYP model also allows calculating the expected number of failures in a period [C;D], knowing that a number m of bursts occurred in a period [A;B] as:

$$E\{X_{|C-D|} \big| m_{|A-B|}\} = \left(\frac{1}{\alpha} + m\right)\frac{\mu_D - \mu_C}{\mu_B - \mu_A + 1}$$

*(Equation 5)*

These examples demonstrate the ability of the invention to introduce a parameter representative of the condition of a pipe within model that predict pipe failures. However, this integration within the LEYP model is provided by means of example only, and pipe condition scores may be integrated within a number of different models of prediction of pipe failure, because the pipe condition score provides a good insight of the instantaneous risk of failure of a pipe.

Coming back to figure 2, the method 200 comprises a sixth step of performing 260 timed predictions of probabilities of pipe failures according to said predictive model.

This step consists in using the predictive model for predicting pipe failures depending upon times. For example, this can be used for determining for each pipe and at various times probabilities of failures of the pipe. For example, the equations to calculate probabilities of failure have been provided below in the example of the LEYP model.

As explained above, the parameterizing of the model using reliable values of pipe conditions allows performing timed predictions of probabilities of pipe failures that are accurate for each pipe of the pipe network, with a limited number of pipe inspections.

Therefore, timed predictions may improve the planning of pipe inspections and renewal in a number of different ways. For example, pipe renewal can be planned in advance in order to prevent pipes to exceed a threshold probability of pipe failure. The pipes may also be ranked by probabilities of failures at a defined time.

The probabilities of failures can be displayed to an operator, so that the operator is able to determine which are the pipe sections to inspect or repaired in priority. This allows adapting pipe inspection plans. This may also be performed by displaying a map with colors corresponding to the expected probabilities of failure of the pipes.

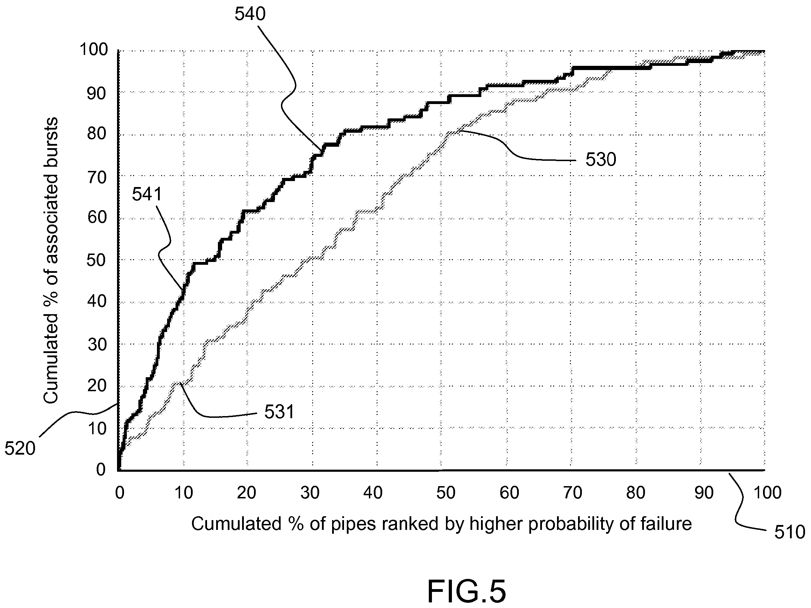

The figure 5 represents the relationship between predicted and actual pipe failures, with a model respectively with and without using pipe condition scores.

The curves 530 and 540 represent experimental results using respectively a model without using pipe conditions scores 530, and using pipe conditions scores 540.

For each model, a probability of failure at a time t is calculated for each pipe of a pipe network. The pipes are ranked from the highest to the lowest probabilities of failure. The horizontal axis represents the cumulated percentage of pipes by probabilities of failure: 10% represents the 10% of pipes with the highest probability of failure, 20% the 20% of pipes with the highest probabilities of failure, etc.

The vertical axis 520 represents the cumulated percentages of the total bursts in the network that actually occurred for the selected percentage of pipes with the highest probabilities of failure. For example, the point 531 means that in the model that does not use the pipe conditions scores (i.e. prior art model, for example the LEYP model without integration of pipe condition scores), the 10% of pipes that were determined as having the highest probability of failure accounted for 20% of total bursts. Meanwhile, the point 541 means that, in the model that uses the pipe conditions scores (model according to the invention, for example LEYP model combined with pipe condition scores), the 10% of pipes that were determined as having the highest probability of failure accounted for 40% of total bursts.

More generally, the curves shows that the invention provides a better prediction of the pipes that are likely to fail, provided that the pipes with the highest probabilities of failures account for more bursts when they are calculated using the invention than using prior art models.

The examples described above are given as illustrations of embodiments of the invention, and demonstrate the ability of the invention to provide a reliable timed-based estimation of the probabilities of failure of pipes of a network when inspecting only a subset of the pipes, and thus at a moderate cost. They do not in any way limit the scope of the invention which is defined by the following claims.

**Claims**

1. A computer-implemented method (200) comprising:

   - a first step of clustering (210) pipe sections of a pipe network into a number of classes, based on pipe parameters relative to the structure or to the environment of the pipe sections;
   - and, for each class of said number of classes:

      - a second step of extracting (220) a representative sample of pipes sections of the class;
      - a third step of obtaining (230), for each pipe section sample, one or more pipe condition scores determined by a condition assessment procedure;
      - a fourth step of performing (240) an estimation of one or more pipe condition scores for pipe sections that do not belong to the sample based on said pipe parameters, said estimation being parameterized with the pipe condition scores and pipe parameters of the pipe sections of the sample extracted at the second step;
      - a fifth step of parameterization (250) of a predictive model of probabilities of pipe failures according to pipe condition scores;
      - a sixth step of performing (260) timed predictions of probabilities of pipe failures according to said predictive model.

   wherein for each class of said number of classes, the fourth step (240) comprises training a supervised machine learning model fed with the pipe condition scores and pipe parameters of the pipe sections of the sample corresponding to this class.

2. The computer-implemented method of claim 1, wherein the predictive model is a probabilistic model that predicts the probability of failure of a
pipe as a function of at least a history of past failures, the age of the pipe, and external factors.

3. The computer-implemented method of claim 2, wherein the pipe condition scores are integrated as external factors.

4. The computer-implemented method of one of claims 2 to 3, depending upon claim 2, wherein the predictive model of probabilities of pipe failures (430) consists in a combination of:

   ◦ a predictive function of probabilities of failures according to time (410) parameterized with said at least one parameters;
   ◦ an instantaneous probability of failure (420) determined based at least on pipe conditions scores of each pipe.

5. The computer-implemented method of one of claims 1 to 4, wherein said number of classes is a predefined number of classes, and the first step comprises the application of a Gaussian Mixture Model, GMM, to the pipes for clustering the pipe sections into said predefined number of classes.

6. The computer-implemented method according to any one of claims 1 to 5, wherein the second step of extracting the sample comprises:

   - a seventh step of initializing a set of candidate samples of pipe sections;
   - an eighth step of iteratively modifying said set of candidate samples using:

      ◦ a genetic algorithm based on an objective function comprising a minimization of the difference of average pipe parameters of the pipe sections of the sample, and
      ◦ the average pipe parameters of the pipe sections of the class;

   - a ninth step of selecting the candidate sample that optimizes said objective function.

7. The computer-implemented method according to any one of claims 1 to 6, wherein the size of each sample is negatively correlated with the homogeneity of each corresponding class.

8. The computer-implemented method of one of claims 1 to 7, wherein the condition assessment procedure of the third step is chosen in a group comprising one or more of:

- an analysis of an electromagnetic flux applied to the pipe section;
- an acoustical analysis of the pipe section;
- the extraction, and analysis in a laboratory of a sample of the pipe section;

and wherein each of the condition assessment procedure provides pipe condition scores at the same scale.

9. The computer-implemented method of claim 8, wherein the condition assessment procedures provide two or more pipe condition scores corresponding to different parts of pipe sections and chosen in a group comprising:

- an inner coating condition score;
- an outer coating condition score;
- a joint condition score.

10. The computer-implemented method of claim 9, wherein a single pipe condition score is obtained from the two or more pipe condition scores corresponding to different parts of pipe sections, using a weighted or orthogonal sum.

11. The computer-implemented method of one of claims 8 to 10, wherein the one or more pipe condition scores are associated with one or more reliability indexes indicating the precision of the pipe condition scores.

12. A computer program product, stored on a non-transitory computer-readable medium, said computer program product comprising code instructions for executing a method according to any one of claims 1 to 11.

13. A device comprising a processor configured to execute a method according to any one of claims 1 to 11.


**Patentansprüche**

1. Computerimplementiertes Verfahren (200), umfassend:

- einen ersten Schritt eines Clusterns (210) von Rohrabschnitten eines Rohrnetzes in eine Anzahl von Klassen, basierend auf Rohrparametern in Bezug auf die Struktur oder auf die Umgebung der Rohrabschnitte;
- und für jede Klasse der genannten Anzahl von Klassen:

- einen zweiten Schritt eines Extrahierens (220) einer repräsentativen Probe von Rohrabschnitten der Klasse;
- einen dritten Schritt eines Erlangens (230), für jede Rohrabschnittsprobe, einer oder mehrerer Rohrzustandsbewertungen, die durch ein Zustandsbeurteilungsverfahren bestimmt werden;
- einen vierten Schritt eines Durchführens (240) einer Schätzung einer oder mehrerer Rohrzustandsbewertungen für Rohrabschnitte, die nicht zu der Probe gehören, basierend auf den Rohrparametern, wobei die Schätzung mit den Rohrzustandsbewertungen und den Rohrparametern der Rohrabschnitte der in dem zweiten Schritt extrahierten Probe parametriert werden;
- einen fünften Schritt eines Parametrierens (250) eines prädiktiven Wahrscheinlichkeitsmodells von Rohrausfällen gemäß den Rohrzustandsbewertungen;
- einen sechsten Schritt eines Durchführens (260) zeitlicher Vorhersagen von Wahrscheinlichkeiten von Rohrausfällen gemäß dem prädiktiven Modell;

wobei der vierte Schritt (240) für jede Klasse der Anzahl von Klassen ein Trainieren eines überwachten Modells maschinellen Lernens umfasst, das mit den Rohrzustandsbewertungen und den Rohrparametern der Rohrabschnitte der dieser Klasse entsprechenden Probe gespeist wird.

2. Computerimplementiertes Verfahren nach Anspruch 1, wobei das prädiktive Modell ein probabilistisches Modell ist, das die Ausfallwahrscheinlichkeit eines Rohrs abhängig von mindestens einer Historie vergangener Ausfälle, dem Alter des Rohrs und externen Faktoren vorhersagt.

**3.** Computerimplementiertes Verfahren
nach Anspruch 2, wobei die Rohrzustandsbewertungen als externe Faktoren integriert werden.

**4.** Computerimplementiertes Verfahren nach einem der Ansprüche 2 bis 3, abhängig von Anspruch 2, wobei das prädiktive Wahrscheinlichkeitsmodell von Rohrleitungsausfällen (430) aus einer Kombination von Folgendem besteht:

o eine prädiktive Funktion von Ausfallwahrscheinlichkeiten abhängig von der Zeit (410), die mit dem mindestens einen Parameter parametriert ist;
o eine momentane Ausfallwahrscheinlichkeit (420), die zumindest basierend auf Rohrzustandsbewertungen für jedes Rohr bestimmt wird.

**5.** Computerimplementiertes Verfahren nach einem der Ansprüche 1 bis 4, wobei die Anzahl von Klassen eine vordefinierte Anzahl von Klassen ist und der erste Schritt die Anwendung eines Gaußschen Mischmodells, GMM, auf die Rohre umfasst, um die Rohrabschnitte in die vordefinierte Anzahl von Klassen zu clustern.

**6.** Computerimplementiertes Verfahren nach einem der Ansprüche 1 bis 5, wobei der zweite Schritt eines Extrahierens der Probe Folgendes umfasst:

- einen siebten Schritt eines Initialisierens eines Satzes von Kandidatenproben von Rohrabschnitten;
- einen achten Schritt eines iterativen Modifizierens des Satzes von Kandidatenproben unter Verwendung von Folgendem:

∘ einem genetischen Algorithmus, der auf einer Zielfunktion basiert, umfassend eine Minimierung der Differenz von durchschnittlichen Rohrparametern der Rohrabschnitte der Probe, und
∘ die durchschnittlichen Rohrparameter der Rohrabschnitte der Klasse;

- einen neunten Schritt eines Auswählens der Kandidatenprobe, die die Zielfunktion optimiert.

**7.** Computerimplementiertes Verfahren nach einem der Ansprüche 1 bis 6, wobei die Größe jeder Probe negativ mit der Homogenität jeder entsprechenden Klasse korreliert ist.

**8.** Computerimplementiertes Verfahren nach einem der Ansprüche 1 bis 7, wobei das Zustandsbewertungsverfahren des dritten Schritts ausgewählt ist aus einer Gruppe, umfassend eines oder mehrere von Folgenden:

- eine Analyse eines elektromagnetischen Flusses, der auf den Rohrabschnitt angewandt wird;
- eine akustische Analyse des Rohrabschnitts;
- die Extraktion und die Analyse einer Probe des Rohrabschnitts in einem Labor; und wobei jedes von dem Zustandsbewertungsverfahren Rohrzustandsbewertungen auf der gleichen Skala bereitstellt.

**9.** Computerimplementiertes Verfahren nach Anspruch 8, wobei die Zustandsbewertungsverfahren zwei oder mehrere Rohrzustandsbewertungen bereitstellen, die verschiedenen Teilen von Rohrabschnitten entsprechen und ausgewählt sind aus einer Gruppe, umfassend:

- eine Innenbeschichtung-Zustandsbewertung;
- eine Außenbeschichtung-Zustandsbewertung;
- eine gemeinsame Zustandsbewertung.

**10.** Computerimplementiertes Verfahren nach Anspruch 9, wobei eine einzige Rohrzustandsbewertung aus den zwei oder mehr Rohrzustandsbewertungen, die verschiedenen Teilen von Rohrabschnitten entsprechen, unter Verwendung einer gewichteten oder orthogonalen Summe erlangt wird.

**11.** Computerimplementiertes Verfahren nach einem der Ansprüche 8 bis 10, wobei der eine oder die mehreren Rohrzustandsbewertungen mit einem oder mehreren Zuverlässigkeitsindizes assoziiert sind, die die Genauigkeit der Rohrzustandsbewertungen angeben.

**12.** Computerprogrammprodukt, das auf einem nicht-transitorischen computerlesbaren Medium gespeichert ist, das Computerprogrammprodukt umfassend Codeanweisungen zum Ausführen eines Verfahrens nach einem der Ansprüche 1 bis 11.

**13.** Vorrichtung, umfassend einen Prozessor, der konfiguriert ist, um ein Verfahren nach einem der Ansprüche 1 bis 11 auszuführen.

**Revendications**

**1.** Procédé mis en œuvre par ordinateur (200), comprenant :

- une première étape consistant à regrouper (210) des sections de canalisation d'un réseau de canalisations en un nombre de classes, sur la base de paramètres de canalisation relatifs à la structure ou à l'environnement des sections de canalisation ;
- et, pour chaque classe dudit nombre de classes :

  - une deuxième étape consistant à extraire (220) un échantillon représentatif des sections de canalisation de la classe ;
  - une troisième étape consistant à obtenir (230), pour chaque échantillon de sections de canalisation, un ou plusieurs scores d'état de canalisation déterminés par une procédure d'évaluation d'état ;
  - une quatrième étape consistant à effectuer (240) une estimation d'un ou plusieurs scores d'état de canalisation pour les sections de canalisation qui n'appartiennent pas à l'échantillon, sur la base desdits paramètres de canalisation, ladite estimation étant paramétrée avec les scores d'état de canalisation et les paramètres de canalisation des sections de canalisation de l'échantillon extrait à la deuxième étape ;
  - une cinquième étape consistant à paramétrer (250) un modèle prédictif des probabilités de défaillance de canalisation en fonction des scores d'état de canalisation ;
  - une sixième étape consistant à effectuer (260) des prédictions temporelles des probabilités de défaillance de canalisation en fonction dudit modèle prédictif.

dans lequel, pour chaque classe dudit nombre de classes, la quatrième étape (240) comprend le fait d'entraîner un modèle d'apprentissage automatique supervisé alimenté par les scores d'état de canalisation et les paramètres de canalisation des sections de canalisation de l'échantillon correspondant à cette classe.

**2.** Procédé mis en œuvre par ordinateur selon la revendication 1, dans lequel le modèle prédictif est un modèle probabiliste qui prédit la probabilité de défaillance d'une canalisation en fonction d'au moins un historique des défaillances passées, de l'âge de la canalisation, et de facteurs externes.

**3.** Procédé mis en œuvre par ordinateur selon la revendication 2, dans lequel les scores d'état de canalisation sont intégrés en tant que facteurs externes.

**4.** Procédé mis en œuvre par ordinateur selon l'une quelconque des revendications 2 à 3, lorsqu'elle dépend de la revendication 2, dans lequel le modèle prédictif des probabilités de défaillance de canalisation (430) consiste en une combinaison de :

  ∘ une fonction prédictive des probabilités de défaillance en fonction du temps (410), paramétrée avec ledit au moins un paramètre ;
  ∘ une probabilité instantanée de défaillance (420) déterminée sur la base au moins des scores d'état de canalisation de chaque canalisation.

**5.** Procédé mis en œuvre par ordinateur selon l'une quelconque des revendications 1 à 4, dans lequel ledit nombre de classes est un nombre prédéfini de classes, et la première étape comprend l'application d'un modèle de mélange gaussien, GMM, aux canalisations afin de regrouper les sections de canalisation dans ledit nombre prédéfini de classes.

**6.** Procédé mis en œuvre par ordinateur selon l'une quelconque des revendications 1 à 5, dans lequel la deuxième étape d'extraction de l'échantillon comprend :

- une septième étape consistant à initialiser un ensemble d'échantillons candidats de sections de canalisation ;
- une huitième étape consistant à modifier de manière itérative ledit ensemble d'échantillons candidats, à l'aide des éléments suivants :

    ◦ un algorithme génétique basé sur une fonction objectif comprenant une minimisation de la différence entre les paramètres de canalisation moyens des sections de canalisation de l'échantillon, et
    ◦ les paramètres de canalisation moyens des sections de canalisation de la classe ;

- une neuvième étape consistant à sélectionner l'échantillon candidat qui optimise ladite fonction objectif.

7. Procédé mis en œuvre par ordinateur selon l'une quelconque des revendications 1 à 6, dans lequel la taille de chaque échantillon est corrélée négativement avec l'homogénéité de chaque classe correspondante.

8. Procédé mis en œuvre par ordinateur selon l'une quelconque des revendications 1 à 7, dans lequel la procédure d'évaluation d'état de la troisième étape est choisie dans un groupe comprenant un ou plusieurs des éléments suivants :

- une analyse d'un flux électromagnétique appliqué à la section de canalisation ;
- une analyse acoustique de la section de canalisation ;
- l'extraction, et l'analyse en laboratoire d'un échantillon de la section de canalisation ;

et dans lequel chacune des procédures d'évaluation d'état fournit des scores d'état de canalisation à la même échelle.

9. Procédé mis en œuvre par ordinateur selon la revendication 8, dans lequel les procédures d'évaluation d'état fournissent deux scores d'état de canalisation ou plus correspondant à différentes parties de sections de canalisation et choisis dans un groupe comprenant :

- un score d'état de revêtement intérieur ;
- un score d'état de revêtement extérieur ;
- un score d'état de raccords.

10. Procédé mis en œuvre par ordinateur selon la revendication 9, dans lequel un score d'état de canalisation unique est obtenu à partir des deux scores d'état de canalisation ou plus correspondant à différentes parties de sections de canalisation, à l'aide d'une somme pondérée ou orthogonale.

11. Procédé mis en œuvre par ordinateur selon l'une quelconque des revendications 8 à 10, dans lequel le ou les scores d'état de canalisation sont associés à un ou plusieurs indices de fiabilité indiquant la précision des scores d'état de canalisation.

12. Produit-programme informatique, stocké sur un support non transitoire lisible par ordinateur, ledit produit-programme informatique comprenant des instructions de code pour exécuter un procédé selon l'une quelconque des revendications 1 à 11.

13. Dispositif comprenant un processeur configuré pour exécuter un procédé selon l'une quelconque des revendications 1 à 11.

FIG.1

210 — | Clustering pipes into classes |

220 — | Extracting representative sample of each class |

230 — | Obtaining pipe condition scores for pipes in the sample |

240 — | Estimating pipe condition scores for pipes that do not belong to sample |

250 — | Parametrizing a model of timed predictions of probabilities of pipe failure |

260 — | Performing predictions of probabilities of pipe failure |

200

# FIG.2

FIG.3

FIG.4

FIG.5

**EP 3 899 522 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4998208 A
- US 2019303791 A
- US 2018300639 A
- WO 2014060655 A

**Non-patent literature cited in the description**

- *Journal de la Société Française de Statistique*, vol. 155 (3), 62-77
- Water Science and Technology. *Water Supply*, vol. 12 (5), 674-682